# EUROPEAN PATENT SPECIFICATION

(11) **EP 3 120 797 B1**
(45) Date of publication and mention of the grant of the patent: **04.09.2019**
(21) Application number: 16180848.0
(22) Date of filing: 22.07.2016
(51) Int. Cl.: A61B 34/10, A61B 17/17

(54) **METHOD FOR IDENTIFYING THE OPTIMAL DIRECTION AND MAXIMUM DIAMETER OF A PEDICLE SCREW**
VERFAHREN ZUR IDENTIFIZIERUNG DER OPTIMALEN RICHTUNG UND DES MAXIMALEN DURCHMESSERS EINER PEDIKELSCHRAUBE
PROCÉDÉ PERMETTANT D'IDENTIFIER LA DIRECTION OPTIMALE ET DIAMÈTRE MAXIMAL D'UNE VIS PÉDICULAIRE

(30) Priority: 22.07.2015 IT UB20152877
(43) Date of publication of application: 25.01.2017
(73) Proprietor: Techno Design S.r.l., 84091 Battipaglia (SA) (IT); Naddeo, Alessandro, 84121 Salerno (SA) (IT); Cataldo, Emilio, 84091 Battipaglia (SA) (IT); Naddeo, Francesco, 84131 Salerno (SA) (IT); Cappetti, Nicola, 84090 Montacorvino Pugliano (SA) (IT); Ruggieri, Roberto, 63074 San Benedetto del Tronto (AP) (IT)
(72) Inventor: Naddeo, Alessandro, 84121 Salerno (SA) (IT); Cataldo, Emilio, 84091 Battipaglia (SA) (IT); Naddeo, Francesco, 84131 Salerno (SA) (IT); Cappetti, Nicola, 84090 Montecorvino Pugliano (SA) (IT)
(74) Representative: Fezzardi, Antonio

(56) References cited:
- WO-A2-2005/081863
- WO-A2-2008/038284
- CN-B- 103 099 680
- US-A1- 2004 240 715
- G F Solitro ET AL: "ANALYTICAL METHOD TO REDUCE PEDICLE SCREW COMPLICATIONS Discover more categories", Las Vegas, 1 April 2015 (2015-04-01), XP055289228, Retrieved from the Internet: URL:https://www.academia.edu/17047543/ANAL YTICAL_METHOD_TO_REDUCE_PEDICLE_SCREW_COMP LICATIONS_G.F._Solitro_and_F._Amirouche_Or thopaedic_Research_Society_Annual_Meeting_ Las_Vegas_March_29_April_1_2015 [retrieved on 2016-07-18] & Giovanni F Solitro ET AL: "Analytical Method to Reduce Pedicle Screw Complications", , 23 March 2015 (2015-03-23), XP055289235, prgmobileapps.com/ Retrieved from the Internet: URL:http://images.google.de/imgres?imgurl= http%3A%2F%2Ffiles.abstractsonline.com%2F% 25255CCTRL%25255C6f%25255C4%25255Cd7d%2525 5C0b6%25255Cd54%25255C4f4%25255C099%25255C e95%25255C1f7%25255C267%25255C47f%25255C02 %25255Cg2251_1.jpg&imgrefurl=http%3A%2F%2F prgmobileapps.com%2FAppUpdates%2Fors2015%2 FAbstrac

## Description

The present invention relates to the sector of implements for surgical use, and in particular has the main purpose of identifying, with the aid of a computer and an x-ray, or of a three-dimensional reconstruction by means of CT (Computed Tomography) or NMR (Nuclear Magnetic Resonance), for a patient, generated by a computer, hence without ever coming into contact *in vivo* with the patient, the optimal direction and the maximum diameter of a pedicle screw so that the screw will not come out of the pedicle in operations of pedicle arthrodesis. The aim of the method according to the invention is hence precisely to obtain the optimal direction of intra-pedicle perforation in the condition of maximum bone material around the axis of the hole, all starting from a definite point P that will be used by a computer for producing in an industrial way a perforating template that can be used in spine surgery.

The basic components of the vertebral column of a human being are the vertebrae, which are set on top of one another along the entire vertebral column and are provided, inside them, with so-called vertebral foramina, which form a kind of canal that receives within it the medulla spinalis (spinal cord), the most important part of the central nervous system.

The vertebral motor unit is, as any part of the body, exposed to the possibility of degenerative pathological conditions that may cause a structural disorder.

In the cases of pathological conditions in which the correction through appropriate devices or physiotherapy is not sufficient for cure, it is indispensable to carry out an operation known as "pedicle arthrodesis".

Pedicle arthrodesis is a surgical technique that consists in fixation of one or more joints of the vertebral column. Its most widespread application envisages the use of implants, and for this reason is also known as "instrumented arthrodesis".

Usually, the operation is performed by means of implants to be fixed to the vertebral column by hooking them to pedicle screws anchored to the vertebrae. The screws are inserted by previously making in the vertebra two lateral holes, using a surgical perforating implement.

The screws, however, if not positioned correctly, might not find a solid and correct location and risk not sinking into the spongy bone tissue, causing, for example, failure or fracture of the cortical tissue or, even, causing caving thereof in the direction of the bone marrow or in the direction of the main blood vessels, hence bringing about irreversible damage and even death of the patient.

Consequently, the pedicle must be perforated in the so-called points of maximum bone material, namely, in the most solid points, and hence the points that provide the greater pullout strength, according to the optimal direction in space.

WO 2005/081863 A2 discloses a fully automatic method for planning the placement of a pedicle screw, where the computer will determine the entry point as well as the direction of the screw without requiring or permitting user interaction.

US 2004/240715 A1 discloses a method for planning the placement of a pedicle screw where the user must define the relevant cross section (the isthmus of the pedicle) and the user must also define the entry point, upon which the method determines the maximum diameter. The person skilled in the branch will be able to obtain a better understanding of the method according to the present invention from the ensuing description and the attached drawings, wherein:

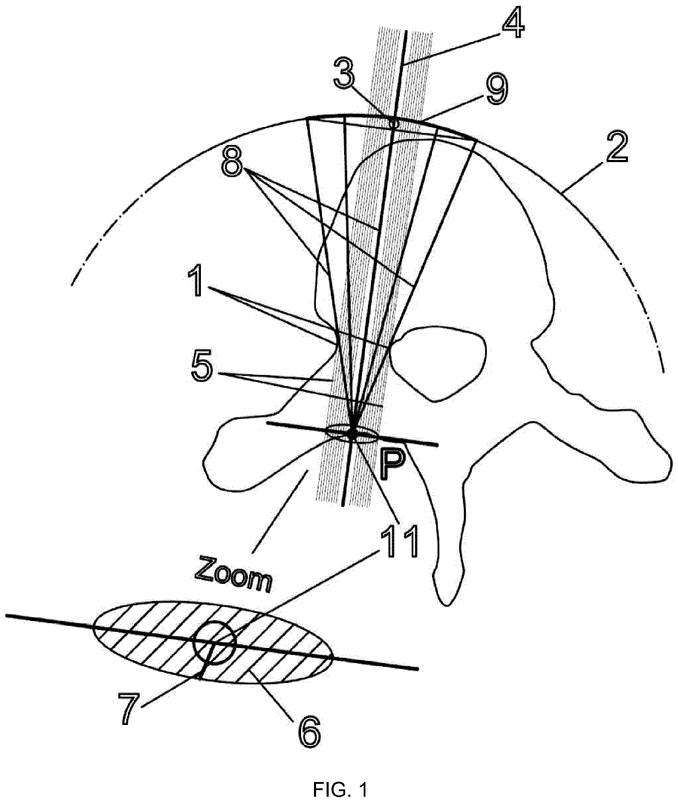

Figure 1 is a two-dimensional schematic representation from above that shows how to apply the method on an x-ray, or on a three-dimensional reconstruction by means of CT or NMR of a patient, generated by a computer at the section of a vertebra for definition of the optimal perforating direction, for insertion of a pedicle screw (10); and

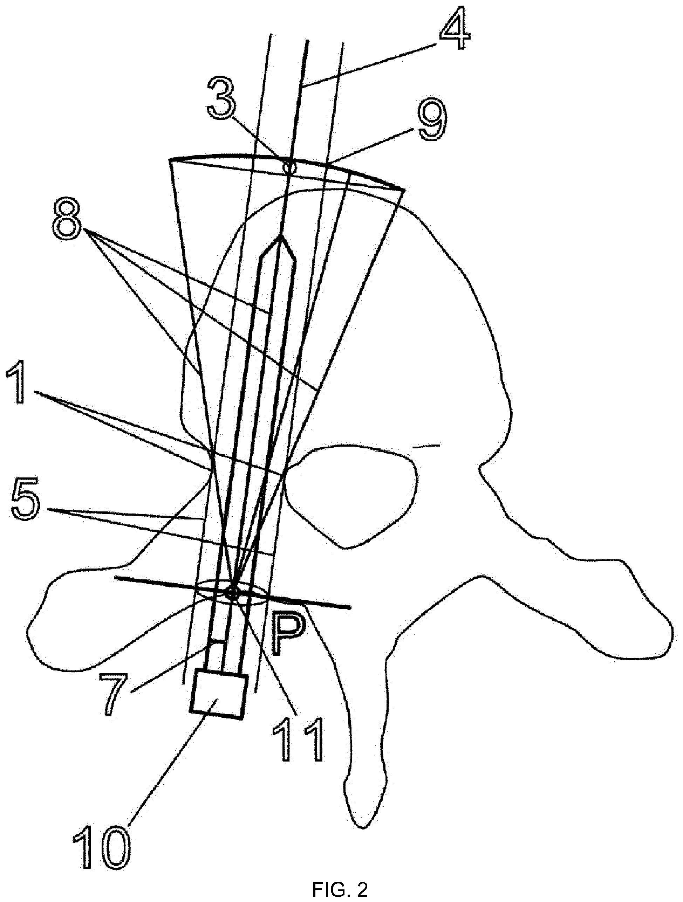

Figure 2 is a top view that shows how, after applying the method according to the present invention, represented on an x-ray, or on a three-dimensional reconstruction by means of CT or NMR, generated by a computer, is a pedicle screw (10) that has as diameter the minimum distance (7) between the point P (11) and the perimeter of the base (6) of a prismatic solid (5) that will be specified in what follows.

The prior art so far available envisages different methods for evaluation of an x-ray, or a three-dimensional reconstruction by means of CT or NMR, of the patient, generated by a computer, of the exact points in which to make the holes for the subsequent sizing of the screws and their consequent application, as emerges from the patent document No. EP173487. This method in fact serves for the design of pedicle screws that can be used in spinal surgery.

The method according to the present invention instead, uses a *problem-solution approach* that differs from the patent document No. EP173487 in so far as the calculation of the maximum diameter and of the optimal direction that the pedicle screw (10) can assume so that it will not come out of the pedicle is carried out on a three-dimensional reconstruction by means of CT or NMR, of the patient, generated by a computer, all starting from a point (P) chosen on the pedicle by the person analysing the three-dimensional reconstruction by means of CT or NMR, hence starting from a choice that exploits and thus takes into account the human experience of the person analysing the three-dimensional reconstruction by means of CT or NMR.

Instead, the methodology described in the patent document No. EP173487, carries out sizing of the pedicle screw starting from the entire pedicle, with the risk of committing an error, which - even though it may amount to just a few millimetres - would not effectively yield an optimal result on the sizing of the pedicle screws.

Another known method is the use of implants, such bars or plates, provided with guide holes. The guide holes, unfortunately, provide the reference point in which to insert the screw, but do not enable perfect orientation of the screw once inserted in the guide.

None of the existing methods enables identification with maximum precision of the correct position and, above all, the optimal direction in which to orient the pedicle screw, and its corresponding sizing.

The present invention has the purpose of identifying, with the aid of a computer and on a three-dimensional reconstruction by means of CT or NMR, of the patient, generated by a computer, hence without ever coming into contact *in vivo* with the patient, the optimal direction and the maximum diameter of a pedicle screw so that the screw will not come out of the pedicle in operations of pedicle arthrodesis.

In what follows, for brevity of exposition, reference will be made to the use of the device on x-rays, but it is to be understood that the method that is claimed is applied on three-dimensional reconstructions using CT or NMR.

The main purpose of the present invention is precisely to provide the optimal direction of intra-pedicular perforation in the condition of maximum bone material around the axis of the hole, all starting from a point that we shall denote by P, which will be used by a computer for producing industrially a perforating template that can be used in spine surgery.

In this connection, the method forming the subject of the present invention comprises the steps described hereinafter.
- First of all, on an x-ray of the patient, generated by a computer, after identification of the three characteristic planes of the vertebra, i.e., the sagittal plane, the coronal plane, and the transverse plane, a point P (11) of entry on the outer surface, at the height of the pedicle, is chosen, without ever coming into contact *in vivo* with the patient.
- There is then generated, by a computer, a spherical cap (2) of centre P (11) and with any radius.
- Generating, by means of a computer, the proper sheaf of straight lines (8) passing through the centre P (11), which intersect the cap (2).
- Eliminating, among the straight lines generated, those intersecting the walls of the pedicle leaving only those fully contained in the pedicle chosen for the perforation, defining, this way, the minimum solid angle that crosses the pedicle and so projects the shape of the available three-dimensional space for the insertion of the screw on the geometric entity more consonant with the case, i.e. the spherical cap (2), which is the geometric element subtending the solid angle.
- Next, all the points of the straight lines (8) intersecting the spherical cap (2) that will constitute the resulting surface (9) are selected by a computer.
- The centroid (3) of the points of intersection of the straight lines (8) with the cap (2) that constitute the resulting surface (9) is calculated by a computer (Figure 1).
- At this point, a straight line (4) passing through the centroid (3) of the resulting surface (9) and the point P (11) is selected by a computer.
- The plane perpendicular to the straight line (4) passing through the point P (11) is created.
- A prismatic solid (5) limited by the tangency of the intra-pedicular section of passage (Figure 1) is generated by a computer.
- The base (6) of the prismatic solid (5) is then selected by a computer.
- The minimum distance (7) between the point P (11) and the perimeter of said base (6) is calculated, said distance (7) representing the maximum radius that the pedicle screw (10) can have (Figure 1).
The straight line (4) passing through the centroid (3) of the resulting surface (9) and the point P (11) generated by a computer represents, on an x-ray of the patient, generated by a computer, the optimal direction that the pedicle screw (10) can have so that it does not come out of the pedicle (Figures 1, 2).

This method is used by a computer for producing in an industrial way an instrument used in the field of pedicle arthrodesis, i.e., a template personalized on the patient for spine surgery, aimed at directional perforation and having a V-shaped structure.

This template - expressly made for a vertebra of a patient on the basis of an x-ray - rests firmly on three points and is characterized by two tubes having the function of guiding the tip of the surgical perforating implement so that the hole is made in the right position, and with the tip of the surgical perforating implement oriented and guided with the right inclination and in the right direction.

The aforesaid perforating template is characterized by a V-shaped support, with which the internal angle of the V adheres to the spinous process of the vertebra perfectly adhering to the latter.

Application of the method according to the present invention, in combination with the use of a personalized innovative template aimed at avoiding the well-known and serious errors of positioning of the implants, which very frequently cause irreversible complications for the patient, guarantees an even more careful and scrupulous precision of the optimal direction, based upon scientific geometrical rigour and upon mechanical supporting devices, as well as guaranteeing a perfect sizing of the pedicle screws.

## Claims

1. A method for extracorporeal identification of the optimal direction and maximum diameter of a pedicle screw so that the screw will not come out of the pedicle in operations of pedicle arthrodesis, **characterized in that** it comprises the following steps:
- choosing a point P (11) of entry on the outer surface at the height of the pedicle, on a three-dimensional reconstruction by means of CT or NMR of a patient, generated by a computer;
- generating, by means of a computer, a spherical cap (2) with centre P (11) and any radius;
- generating, by means of a computer, the proper sheaf of straight lines (8) passing through the centre P (11), which intersect the cap (2) and are contained in the pedicle chosen for the perforation;
- selecting, by means of a computer, all the points of the straight lines (8) intersecting the spherical cap (2) that will constitute a resulting surface (9);
- calculating, by means of a computer, the centroid (3) of the points of intersection of the straight lines (8) with the cap (2) that constitute the resulting surface (9);
- selecting, by means of a computer, a straight line (4) passing through the centroid (3) of the resulting surface (9) and the point P (11);
- tracing, by means of a computer, the plane perpendicular to the straight line (4) passing through the point P (11);
- generating, by means of a computer, a prismatic solid (5) limited by the tangency (1) of the intra-pedicular section of passage, wherein said prismatic solid (5) extends in parallel to the straight line (4);
- selecting the base (6) of the prismatic solid (5); and
- identifying, by means of a computer, the minimum distance (7) between the point P (11) and the perimeter of said base (6), where said minimum distance (7) represents the maximum radius that the pedicle screw (10) can have.

2. The method according to Claim 1, **characterized in that** the straight line (4) passing through the centroid (3) of the resulting surface (9) and the point P (11), generated by a computer, is the optimal direction that the pedicle screw (10) can have so that it will not come out of the pedicle, identified on a three-dimensional reconstruction by means of CT or NMR of said patient, generated by a computer.

3. The method according to one or more of the preceding claims, **characterized in that** said straight line (4) passing through the centroid (3) of the resulting surface (9) and the point P (11), which represents the optimal direction that the pedicle screw (10) can have so that it will not come out of the pedicle, is used as axis of a directional cylindrical guide tube of a directional perforating template for spine surgery.

4. The method according to the preceding claim, **characterized in that** said template is made specifically for the patient on which said three-dimensional CT or NMR reconstruction has been carried out.

## Patentansprüche

1. Verfahren für extrakorporale Identifikation der optimalen Richtung und des maximalen Durchmessers einer Pedikelschraube, so dass die Schraube bei Operationen von Pedikelarthrodese nicht aus dem Pedikel austritt, **dadurch gekennzeichnet, dass** es die folgenden Schritte umfasst:
- Auswählen eines Punktes P (11) des Eintritts an der äußeren Fläche auf Höhe des Pedikels auf einer dreidimensionalen Rekonstruktion mittels CT oder NMR eines Patienten, die von einem Computer erzeugt wird;
- Erzeugen, mittels eines Computers, einer Kugelhaube (2) mit Zentrum P (11) und einem beliebigen Radius;
- Erzeugen, mittels eines Computers, der angemessenen Garben gerader Linien (8), die durch das Zentrum P (11) verlaufen, die die Haube (2) schneiden und in dem Pedikel, das für die Perforation ausgewählt wurde, enthalten sind;
- Auswählen, mittels eines Computers, aller Punkte der geraden Linien (8), die die Kugelhaube (2) schneiden, die eine resultierende Fläche (9) konstituieren werden;
- Berechnen, mittels eines Computers, des Schwerpunktes (3) der Schnittpunkte der geraden Linien (8) mit der Haube (2), die die resultierende Fläche (9) konstituieren;
- Auswählen, mittels eines Computers, einer geraden Linie (4), die durch den Schwerpunkt (3) der resultierenden Fläche (9) und den Punkt P (11) verläuft;
- Verfolgen, mittels eines Computers, der Ebene senkrecht zu der geraden Linie (4), die durch den Punkt P (11) verläuft;
- Erzeugen, mittels eines Computers, eines prismatischen Festkörpers (5), der durch die Tangenz (1) des intrapedikulären Durchgangsabschnitts begrenzt ist, wobei sich der prismatische Festkörper (5) parallel zu der geraden Linie (4) erstreckt;
- Auswählen der Basis (6) des prismatischen Festkörpers (5); und
- Identifizieren, mittels eines Computers, der Mindestentfernung (7) zwischen dem Punkt P (11) und dem Perimeter der Basis (6), wobei die Mindestentfernung (7) den maximalen Radius repräsentiert, den die Pedikelschraube (10) haben kann.

2. Verfahren nach Anspruch 1, **dadurch gekennzeichnet, dass** die gerade Linie (4), die durch den Schwerpunkt (3) der resultierenden Fläche (9) und den Punkt P (11) verläuft, von einem Computer erzeugt, die optimale Richtung ist, die die Pedikelschraube (10) haben kann, so dass sie nicht aus dem Pedikel austritt, identifiziert auf einer dreidimensionalen Rekonstruktion mittels CT oder NMR des Patienten, erzeugt durch einen Computer.

3. Verfahren nach einem oder mehreren der vorstehenden Ansprüche, **dadurch gekennzeichnet, dass** die gerade Linie (4), die durch den Schwerpunkt (3) der resultierenden Fläche (9) und den Punkt P (11) verläuft, die die optimale Richtung repräsentiert, die die Pedikelschraube (10) haben kann, so dass sie nicht aus dem Pedikel austritt, als Achse eines direktionalen zylindrischen Führungsrohrs einer direktionalen perforierenden Vorlage für Wirbelsäulenchirurgie verwendet wird.

4. Verfahren nach dem vorstehenden Anspruch, **dadurch gekennzeichnet, dass** die Vorlage speziell für den Patienten hergestellt wird, an dem die dreidimensionale CT- oder NMR-Rekonstruktion durchgeführt wurde.

## Revendications

1. Procédé d'identification extracorporelle de la direction optimale et du diamètre maximal d'une vis pédiculaire afin que la vis ne sorte pas du pédicule dans des opérations d'arthrodèse de pédicule, **caractérisé en ce qu'**il comprend les étapes suivantes :
- le choix d'un point P (11) d'entrée sur la surface extérieure à la hauteur du pédicule, sur une reconstruction tridimensionnelle par TDM ou RMN d'un patient, générée par un ordinateur ;
- la génération, au moyen d'un ordinateur, d'une calotte sphérique (2) avec le centre P (11) et tout rayon ;
- la génération, au moyen d'un ordinateur, du faisceau correct de droites (8) passant par le centre P (11), qui coupent la calotte (2) et sont contenues dans le pédicule choisi pour la perforation ;
- la sélection, au moyen d'un ordinateur, de tous les points des droites (8) coupant la calotte sphérique (2) qui constitueront une surface résultante (9) ;
- le calcul, au moyen d'un ordinateur, du centroïde (3) des points d'intersection des droites (8) avec la calotte (2) qui constituent la surface résultante (9) ;
- la sélection, au moyen d'un ordinateur, d'une droite (4) passant par le centroïde (3) de la surface résultante (9) et le point P (11) ;
- le traçage, au moyen d'un ordinateur, du plan perpendiculaire à la droite (4) passant par le point P (11) ;
- la génération, au moyen d'un ordinateur, d'un solide prismatique (5) limité par la tangence (1) de la section de passage intrapédiculaire, ledit solide prismatique (5) s'étendant en parallèle à la droite (4) ;
- la sélection de la base (6) du solide prismatique (5) ; et
- l'identification, au moyen d'un ordinateur, de la distance minimale (7) entre le point P (11) et le périmètre de ladite base (6), où ladite distance minimale (7) représente le rayon maximal que la vis pédiculaire (10) peut avoir.

2. Procédé selon la revendication 1, **caractérisé en ce que** la droite (4) passant par le centroïde (3) de la surface résultante (9) et le point P (11), générée par un ordinateur, est la direction optimale que la vis pédiculaire (10) peut avoir afin qu'elle ne sorte pas du pédicule, identifiée sur une reconstruction tridimensionnelle par TDM ou RMN dudit patient, générée par un ordinateur.

3. Procédé selon une ou plusieurs des revendications précédentes, **caractérisé en ce que** ladite droite (4) passant par le centroïde (3) de la surface résultante (9) et le point P (11), qui représente la direction optimale que la vis pédiculaire (10) peut avoir afin qu'elle ne sorte pas du pédicule, est utilisée en tant qu'axe d'un tube de guidage cylindrique directionnel d'un gabarit de perforation directionnel pour une opération chirurgicale de la colonne vertébrale.

4. Procédé selon la revendication précédente, **caractérisé en ce que** ledit gabarit est réalisé spécifiquement pour le patient sur lequel ladite reconstruction TDM ou RMN tridimensionnelle a été réalisée.
